# EUROPEAN PATENT SPECIFICATION

(11) **EP 1 458 725 B1**
(45) Date of publication and mention of the grant of the patent: **21.02.2007**
(21) Application number: 02786991.6
(22) Date of filing: 10.12.2002
(51) Int. Cl.: C07D 493/10, A61K 31/365, A61K 31/335, A01N 43/00, A61P 31/10, C12N 1/14

(54) **A NOVEL SORDARIN DERIVATIVE ISOLATED FROM CULTURE FERMENTATIONS AND FUNCTIONS AS AN ANTIFUNGAL AGENT**
NEUES, AUS KULTURFERMENTIERUNGEN ISOLIERTES SORDARINDERIVAT UND WIRKUNGEN ALS ANTIPILZMITTEL
DERIVE DE SORDARINE OBTENU PAR FERMENTATION DE CULTURES, CONVENANT COMME FONGICIDE

(30) Priority: 14.12.2001 US 340367 P
(43) Date of publication of application: 22.09.2004
(73) Proprietor: Merck & Co., Inc., Rahway, New Jersey 07065-0907 (US); Merck Sharp & Dohme de Espana S.A., 28027 Madrid (ES)
(72) Inventor: GUAN, Ziqiang, Rahway, NJ 07065-0907 (US); HARRIS, Guy, H., Rahway, NJ 07065-0907 (US); JUSTICE, Michael, C., Rahway, NJ 07065-0907 (US); NIELSEN-KAHN, Jennifer, Rahway, NJ 07065-0907 (US); SHASTRY, Mythili, S., Rahway, NJ 07065-0907 (US); BASILIO, Angela, E-28027 Madrid (ES); COLLADO, Javier, E-28027 Madrid (ES); DIEZ, Maria Teresa, E-28027 Madrid (ES)
(74) Representative: Buchan, Gavin MacNicol
(86) International application number: PCT/US2002/039411
(87) International publication number: WO 2003/051889

(56) References cited:
- US-A- 4 436 735
- F.C.ODDS: "Sordarin antifungal agents" EXPERT OPINION ON THERAPEUTIC PATENTS, vol. 11, no. 2, February 2001 (2001-02), pages 283-294, XP002335597

## Description

### FIELD OF INVENTION

The present invention relates to a novel compound derived by fermentation that is useful as an antifungal agent.

### BACKGROUND OF THE INVENTION

Sordarin is an antifungal antibiotic isolated from the mould *Sordaria araneosa* (see GB 1,162,027 and *Helvetica Chimica Acta,* 1971, 51:119-20). Other compounds having the sordarin skeleton have also been reported as antifungal agents. Japanese Kokai J62040292 discloses the compound zofimarin isolated from *Zopfiella marina* sp.; Japanese Kokai J06157582 discloses the compound BE-31405 isolated from *Penicillium* sp.; and SCH57404 is reported in *J. Antibiotics,* 1995, 48:1171-1172. Semi-synthetic sordarin derivatives are reported in PCT Applications WO96/14326 and WO96/14327.

Sordaricin, the aglycone, may be obtained from sordarin by acid hydrolysis (Hauser and Sigg, *Helvetica Chimica Acta,* 1971, 51:119-20). The total synthesis of sordaricin methyl ester is reported in Kato et al., *J. Chem. Soc., Chem. Commun.,* 1993, 1002-1004, which also discloses *o*-methoxymethyl sordaricin methyl ester. The diacetate of 4-deformyl-4-hydroxymethyl sordaricin is disclosed in Mander and Robinson, *J. Org._Chem.,* 1991, 56(11):3395-3601. Neither sordaricin nor the reported derivatives thereof has been shown to have biological activity.

Balkovec et al. (U.S. Patent Nos. 6,040,463 and 6,136,853) discloses a compound of sordarin derivative.

Nielsen-Kahn et al. (U.S. Patent No. 5,972,996) and Tse, Bruno (U.S. Patent No. 5,965,612) disclose a compound of 4-cyano-4-deformylsordarin derivatives.

Sturr et al. (U.S. Patent No. 6,228,622) discloses a compound of C11-hydroxysodarin and a process for producing it using Actinomyces spp, (Merck Culture Collection MA7235) by a biotransformation.

An objective of the present invention is to provide a novel sordarin analog isolated from a fermentation that is potent antifungal agents for general use against pathogens associated with human and agricultural fungal infections.

### SUMMARY OF THE INVENTION

The present invention relates to a compound of formula I 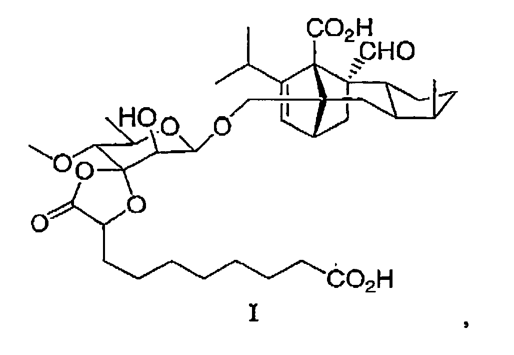 or a pharmaceutically or agriculturally acceptable salt thereof, which is a potent antifungal agent with a broad spectrum of activity, which can be used against pathogens associated with human and agricultural fungal infections. The present invention also include a method for preparing the compound of formula I, a pharmaceutical and agricultural composition containing the compound, its use in the manufacture of a medicament for treatment or prevention of a fungal infection in humans and animal, and a method of controlling fungal infections in plant materials using the compound of formula I.

### BRIEF DESCRIPTION OF THE DRAWINGS

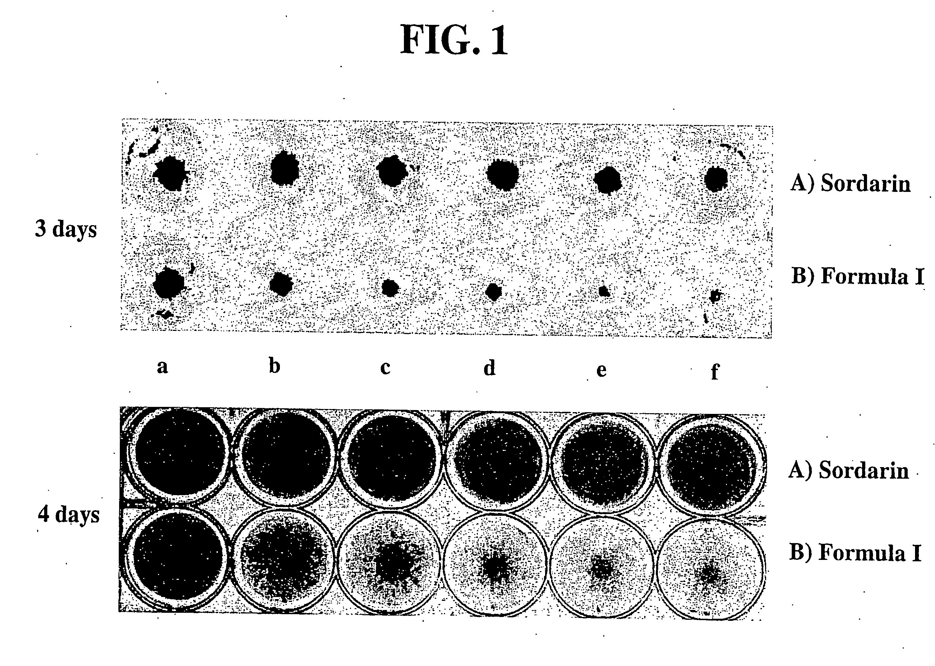

Figure 1. Potato-Dextrose Agar plates dosed with a) 0, b) 1, c) 2, d) 5, e) 10, and f) 20 µg/ml of A) sodarin and B) the compound of formula I then the plates were infected with a suspension of *Botrytis cinerea* photographed at 3 and 4 days.

### DETAILED DESCRIPTION OF THE INVENTION

The present invention relates to a compound of formula I 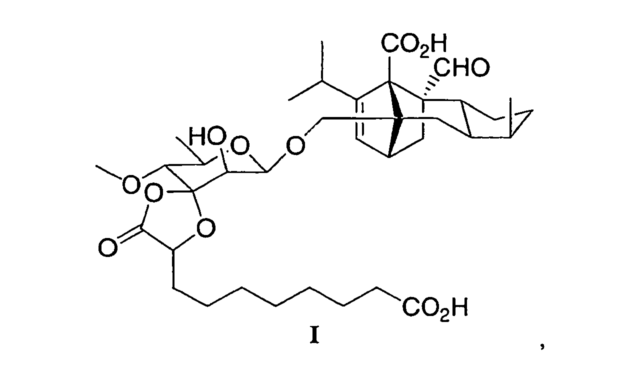 or a pharmaceutically or agriculturally acceptable salt thereof, which is a potent antifungal agent with a broad spectrum of activity, which can be used against pathogens associated with human and agricultural fungal infections.

One aspect of the present invention provides a pharmaceutical composition, which comprises a compound of formula I and a pharmaceutically acceptable carrier.

Another aspect of the present invention provides a pharmaceutical formulation comprising a combination of a compound of formula I and a second therapeutic agent or its pharmaceutically acceptable salt. The second therapeutic agent is a compound selected from the group consisting of an azole, polyene, purine nucleotide inhibitor, pneumocandin derivative, echinocandin derivative, the elongation factor inhibitor, and immunomodulating agent. More particularly, the second therapeutic agent of selected from azoles such as fluconazole, voriconazole, itraconazole, ketoconazole, miconazole, ER 30346, SCH 56592; polyenes such as amphotericin B, nystatin or liposomal and lipid forms thereof such as Abelcet, AmBisome and Amphocil; purine or pyrimidine nucleotide inhibitors such as flucytosine; or polyoxins such as nikkomycins, in particular nikkomycin Z or other chitin inhibitors, elongation factor inhibitors such as sordarin and analogs thereof, mannan inhibitors such as predamycin, bactericidal/permeability-inducing (BPI) protein products such as XMP.97 or XMP.127 or complex carbohydrate antifungal agents and pneumocandin or echinocandin derivatives such as caspofungin and micofungin. A preferred second therapeutic agent is a compound selected from the group consisting of intraconazole, flucytosine, fluconazole, amphotericin B, and caspofungin.

Yet another aspect of the present invention provides an agrochemical composition, which comprises a compound of formula I and an agriculturally acceptable carrier.

Yet another aspect of the present invention provides an agrochemical composition, which comprises a compound of formula I and a second active ingredient selected from the group consisting of herbicides, insecticides, bactericides, nematocides, molluscicides, growth regulators, micronutrients, fertilizers, and fungicides.

Yet another aspect of the present invention provides the use of a compound of formula I for the manufacture of a medicament for the treatment or prevention of a fungal infection in a mammal (including humans).

Said treatment or prevention of fungal infection in a mammal may comprise administering to said mammal therapeutically effective amounts of a compound of formula I and a second therapeutic agent selected from the group consisting of an azole, polyene, purine pyrimidine nucleotide inhibitor, pneumocandin derivative, echinocandin derivative, polyoxins, mannan inhibitors, bacbericidal/permeability inducing protein products, elongation factor inhibitor, and immunomodulating agent.

Yet another aspect of the present invention provides a method for controlling phytopathogenic fungi, which comprises administering to a plant in need of such control therapeutically effective amounts of a compound of formula I.

A further aspect of the present invention provides a method for controlling phytopathogenic fungi, which comprises administering to a plant in need of such control therapeutically effective amounts of a compound of formula I and a second active ingredient selected from the group consisting of herbicides, insecticides, bactericides, nematocides, molluscicides, growth regulators, micronutrients, fertilizers, and fungicides.

As used herein, unless otherwise specified, the following terms have the indicated meanings.

The term "plants" include live plants, foliage, flowers, seeds, fruits, and other materials derived from plants. The term also includes roots of the plant via application of the active ingredient to the soil.

The term "composition", as in pharmaceutical or agricultural composition, is intended to encompass a product comprising the active ingredient(s), and the inert ingredient(s) that make up the carrier, as well as any product which results, directly or indirectly, from combination, complexation, aggregation or other interactions of any two or more of the active ingredient(s) and/or the inert ingredient(s) or from dissociation of one or more of the active ingredient(s) and/or the inert ingredient(s), or from other types of reactions of one or more of the active ingredient(s) and/or the inert ingredient(s).

Suitable salts of a compound of formula I include inorganic base salts such as alkali metal salt (e.g. sodium and potassium salts), ammonium salts, and organic base salts. Suitable organic base salts include amine salts such as tetraalkylammonium (e.g. tetrabutylammonium or trimethylcetylammonium), trialkylamine (e.g. triethylamine), dialkylamine salts (e.g. dicyclohexylamine), optionally substituted benzylamine (e.g. phenylbenzylamine or para-bromobenzylamine), ethanolamine, diethanolamine, N-methylglucosamine, N-methylpiperidine, pyridine and substituted pyridine (e.g. collidine, lutidine, 4-dimethylaminopyridine), and tri(hydroxymethyl)methylamine salts, and amino acid salts (e.g. lysine or arginine salts).

A mammal as used in here includes a human and a warm blooded animal such as a cat, a dog and the like.

The compounds of the present invention are formed by the fermentation of the cultures MF6856 or MF6864.

The culture MF6856 is a strain of *Morinia sp.* (mitosporic fungus) which was isolated from a sample of *Sedum sedifonne* (Jacq.) collected in Sierra Alhamilla, Almería, Spain. The fungus, MF6856 in the culture collection of Merck & Co., Inc., Rahway, N.J., has been deposited under the Budapest Treaty in the culture collection of the American Type Culture Collection on November 14, 2001 at 10801 University Blvd., Manassas, Virginia 20110-2209 and assigned the accession number ATCC No. PTA-3862.

The fungal colonies exhibit the following morphological features in agar culture (color names and codes from Komerup, A. & Wanscher, J.H. (1978). *Methuen Handbook of Colour.* 3rd edition. Eyre, Methuen, London):

Colonies on cornmeal agar (Difco) attaining 65 mm after 21 days at 22°C and 80% humidity; flat, glabrous, hyaline, with a small area (8-10 mm diam.) of white mycelium at the center. Odor and exudates absent. Reverse colorless. Margin regular. Soluble pigments absent.

Colonies on oatmeal agar (Difco) attaining 70 mm after 21 days at 22°C and 80% humidity; flat, texture velvety to slightly floccose; with alternate pinkish white (7A2) and grayish red (7B3) fringes disposed concentrically. Odor and exudates absent. Reverse brownish yellow (5C8). Soluble pigments absent.

Colonies on potato-dextrose agar (Difco) attaining 30 mm after 21 days at 22°C and 80% humidity; rugose with few radial grooves; margin irregular; center velvety, olive grey (3D2) with brown (7E8) exudates, surrounded by a clearer yellowish grey (3B2) wide fringe; colony edge two-colored, with and external white ring and an inner narrow and olive (2E4) fringe. Diffusible light brown (7D7) pigment produced. Odor absents.

Mycelium branched, septate, with hyaline and olive yellow hyphae, 5 µm wide. Conidiomata acervular, globose; conidiophores lining the cavity of the conidioma, simple or branched at the bottom; conidiogenous cells smooth, hyaline, cylindrical, mostly 1 septate, 9.7-19 (13) x 1.9-3 (2.6) µm. Conidia muriform, fusiform, ellipsoid, pyriform, straight to slightly curved, smooth, 19.5-24.7 (22.4) x 6.5 µm, with 6 or 7 transverse septa and 1 or 2 vertical and oblique septa; versicolored, with basal and apical cells hyaline to subhyaline; medium cells (15-17 µm long) brown and concolorous throughout; basal cell conic, frequently with no appendages but sometimes bearing one more or less centric short cellular appendage (rarely two excentric) 3 µm long; hemispherical apical cell crowned with three single cellular appendages, straight or slightly curved, 9-11 (10.4) µm long.

MF6856 is assigned to the genus *Morinia* based on globose acervuli bearing muriform conidia with three single apical appendages. MF6856 exhibits some morphological similarities to the only species of the genus, *M. pestalozzioides* Berl. & Bres. (Guba, E.F. 1961. *Monograph of Monochaetia and Pestalotia.* Harvard University Press: Cambridge, Massachusetts), which has similar conidiomata as well as conidia elliptic fusoid, muriform, versicolored, usually crowned with three apical appendages. However, there are some important differences that preclude MF6856 of being assigned to *M. pestalozzioides.* Thus, MF6856 produces conidia with shorter apical appendages than *M. pestalozzioides* and occasionally with one or two short basal appendages, which are lacking in *M. pestalozzioides.* Moreover, *M. pestalozzioides* conidia are generated on flexuous filiform conidiogenous cells that often remain attached after the conidial secession as a pedicel, while MF6856 conidia are produced on much broader and shorter cylindrical conidiogenous cells that seldom remain attached to the conidial basal cell after secession. Therefore, MF6856 is classified as an undetermined species of *Morinia.*

The culture MF6864 is a strain of *Cladosporium sp.,* which was isolated from a cactus trunk sample collected in Puerto Vargas, Costa Rica. The fungus, MF6864 in the culture collection of Merck & Co., Inc., Rahway, N.J., has been deposited under the Budapest Treaty in the culture collection of the American Type Culture Collection on November 14, 2001 at 10801 University Blvd., Manassas, Virginia 20110-2209 and assigned the accession number ATCC No. PTA-3861.

The fungal colonies exhibit the following morphological features in agar culture (color names and codes from Kornerup, A. & Wanscher, J.H. (1978). Methuen Handbook of Colour. 3rd edition. Eyre, Methuen, London).

Colonies on cornmeal agar (Difco) attaining a diameter of 77 mm after 21 days at 22°C and 80% humidity; flat, texture floccose at the center, but not tightly compressed, olive grey (2F2), becoming less hairy and yellowish grey (2D2, 2B2) towards the edges. Odor and exudates absent. Margin regular, hyaline. Reverse olive-brown (4F6), appearance radiate, with an array of concentric rings around the center. Soluble pigment absents.

Colonies on oatmeal agar (Difco) attaining 75 mm in 21 days at 22°C and 80% humidity; flat with a depression of 5-10 mm diameter in the colony center, which contains a tuff of dark hyphae; texture velvety to floccose; dark grey (2F1) at the center, becoming light grey (2B2) towards the margins. Margin white, regular. Odor and exudates absent. Reverse medium grey (4E1). Soluble pigment absents.

Colonies on potato-dextrose agar (Difco) attaining 80 mm in 21 days at 22°C, 80% humidity; flat, velvety at the center, with alternate dark grey (1F1) and medium grey concentric fringes (1E1), becoming clearer (4B1) and sparse towards the margins, hyaline at the edge. Hyaline exudates produced on clusters of dematiaceous hyphae, which form dark spots irregularly distributed around the colony center. Margin regular. Reverse olive (3F7). Odor absents. Soluble pigment light yellow (3A5).

Vegetative hyphae septate, hyaline, irregular in size, or forming fasciculi of olivaceous hyphae. Conidiophores macronematous, 80-150 µm long, occasionally much longer, 3-5 µm thick, pale to mid olivaceous-brown, smooth or finely roughened. Ramoconidia smooth, pale olivaceous-brown, 15-32 (21.5) x 3-5 (4.5) µm, 0-4 (more frequently 2) septate. Conidiogenous cells smooth, pale olivaceous brown, polyblastic, usually integrated, terminal and intercalary, sympodial, nearly cylindrical, cicatrized, scars prominent. Conidia smooth, pale olivaceous brown to hyaline, ellipsoidal, limoniform, 3-7 (5) x 2-3.5 (2.5) µm 0 septate, with prominent scars.

The combination of macronematous and olivaceous brown conidiophores, presence of ramoconidia, conidiogenous cells integrated, polyblastic, sympodial and conidia in branching chains with protuberant scars, either at each end or just at the base, is characteristic of the genus *Cladosporium* Link ex Fries (anamorphic Mycosphaerellaceae). MF6864 produces long, straight, and brown conidiophores without swellings that resemble those of *C. tenuissimum* Cooke (Fresen.) de Vries., a cosmopolitan fungal species common in tropical countries. Nevertheless, *C. tenuissimum* has a much broader conidial size range than MF6864 (Ellis, M.B. 1976. *More Dematiaceous Hyphomycetes.* Commonwealth Mycological Institute: Kew, UK). The divergence in the conidial size, in addition to the lack of information available on other morphological characters related to conidiophore branching or ramoconidia in *C. tenuissimum,* precludes the unambiguous identification of MF6864 as a strain of this species, and therefore this isolate will be classified as an undetermined species of *Cladosporium.*

### Fermentation Procedure

In general, MF6856 (ATCC No. PTA-3862), identified as *Morinia* sp. and MF6864 (ATCC No. PTA-3861), identified as *Cladosporium* sp. are cultured in an aqueous nutrient medium containing sources of assimilable carbon and nitrogen. For example, the cultures can be grown under submerged aerobic conditions (e.g., shaking culture, submerged culture, etc.). The aqueous medium is preferably maintained at a pH of about 6-8 at the initiation and termination (harvest) of the fermentation process. The desired pH may be maintained by the use of a buffer such as morpholinoethane-sulfonic acid (MES), morpholinopropanesulfonic acid (MOPS), and the like, or by choice of nutrient materials which inherently possess buffering properties.

The preferred source of carbon in the nutrient medium are carbohydrates such as glucose, xylose, galactose, glycerin, starch, sucrose, dextrin, and the like. Other sources which may be included are maltose, rhamnose, raffinose, arabinose, mannose, sodium succinate, and the like.

The preferred sources of nitrogen are yeast extract, meat extract, peptone, gluten meal, cottonseed meal, soybean meal and other vegetable meals (partially or totally defatted), casein hydrolysates, soybean hydrolysates, and yeast hydrolysates, corn steep liquor, dried yeast, wheat germ, feather meal, peanut powder, distiller's solubles, etc., as well as inorganic and organic nitrogen compounds such as ammonium salts (e.g., ammonium nitrate, ammonium sulfate, ammonium phosphate, etc.), urea, amino acids, and the like.

The carbon and nitrogen sources, though advantageously employed in combination, need not be used in their pure form, because less pure materials which contain traces of growth factors and considerable quantities of mineral nutrients, are also suitable for use. When desired, there may be added to the medium mineral salts such as sodium or calcium carbonate, sodium or potassium phosphate, sodium or potassium chloride, sodium or potassium iodide, magnesium salts, copper salts, cobalt salts, and the like. If necessary, especially when the culture medium foams seriously, a defoaming agent, such as liquid paraffin, fatty oil, plant oil, mineral oil or silicone may be added.

As to the conditions for the production of cells in massive amounts, submerged aerobic cultural conditions is one method of culturing the cells. For the production in small amounts, a shaking or surface culture in a flask or bottle is employed. Furthermore, when the growth is carried out in large tanks, it is preferable to use the vegetative forms of the organism for inoculation in the production tanks in order to avoid growth lag in the process of production. Accordingly, it is desirable first to produce a vegetative inoculum of the organism by inoculating a relatively small quantity of culture medium with spores or mycelia of the organism produced in a "slant" and culturing said inoculated medium, also called the "seed medium", and then to transfer the cultured vegetative inoculum aseptically to large tanks. The fermentation medium, in which the inoculum is produced, is generally autoclaved to sterilize the medium prior to inoculation. The pH of the medium is generally adjusted to about 6-7 to the autoclaving step.

Agitation and aeration of the culture mixture may be accomplished in a variety of ways. Agitation may be provided by a propeller or similar mechanical agitation equipment, by revolving or shaking the fermentor, by various pumping equipment, or by the passage of sterile air through the medium. Aeration may be effected by passing sterile air through the fermentation mixture.

The fermentation is usually conducted at a temperature between about 20°C and 30°C, preferably 22-25°C, for a period of about 14-28 days, which may be varied according to fermentation conditions and scales.

Preferred culturing/production media for carrying out the fermentation include the media as set forth in the Examples.

### Isolation and Characterization of the Compound

Compound I is recovered from fermentations of either fungal culture by extraction with methyl ethyl ketone and mixing for several hours at room temperature. The mixture is then filtered and the filtrate concentrated to dryness to contain crude compound of formula I as a residue. Other suitable extraction solvents include ethyl acetate, methanol and acetone. The liquid fermentation broth is extracted by adding an equal volume of methanol and stirring for several hours at room temperature. An immiscible solvent such as methyl ethyl ketone or ethyl acetate is also suitable. The mixture is then filtered to yield a solution of crude compound of formula I. Other suitable extraction solvents include acetone or ethanol.

The product from solid fermentation broth is recovered from the extract by adsorption / elution on an ion exchange resin. The product from liquid fermentations is recovered by solid phase adsorption/elution on polystyrenedivinyl benzene resins. The eluate is then further purified on a wealdy basic ion exchange resin as described for solid fermentations. The product is then further isolated by chromatography, preferably on reverse phase.

The preferred ion exchange adsorption/elution for the capture of compound of formula I from crude fermentation resin is a hydrophilic weakly basic polymer. The crude extract containing compound of formula I is dissolved in a mixture of methanol and water, adjusted to approximately pH 7, and adsorbed onto a bed of ion exchange resin. Compound of formula I is recovered by washing the resin with a higher ionic strength solution or by lowering the pH of the eluant solution. The preferred adsorption / elution resins for the capture of compound of formula I from extractions of liquid fermentations using a water miscible solvent are polystryenedivinylbenzene polymers such as Mitsubishi SP207. Compound of formula I is adsorbed onto the resin at low organic solvent concentrations, high aqueous concentrations, and eluted by washing the resin with an organic solvent such as methanol. High speed countercurrent chromatography may also be used for the initial purification of compound of formula I from methyl ethyl ketone extracts of solid fermentations.

The preferred method for final purification of compound of formula I is reverse phase liquid chromatpgraphy. The stationary phase may be either a C8 or C18 bonded phase. The preferred eluant is an acidified mixture water and acetonitrile or methanol. Compound of formula I can then be recovered by concentration in vacuo or by lyophilization after removal of the acetonitrile or methanol.

The recovered compound can then be further characterized by infrared spectrum, NMR spectrum, and mass spectrum.

### SWORDIII Assay

The natural product sordarin is a tetracyclic diterpene glycoside, which inhibits fungal protein synthesis by impairing the function of eEF2 despite the high degree of amino acid sequence homology exhibited by eEF2 proteins from various eukaryotes. Sordarin and its derivatives bind to the eEF2-ribosome-nucleotide complex in sensitive fungi. Through different *in vitro* assays using purified components from human, yeast and plant cells, it has been shown that the selective sensitivity to sordarin and its derivatives is dependent on fungal eEF2. The fungal specificity of the sordarin makes eEF2 an attractive anti-infective target. (J Biol Chem. 1998, Feb 6; 273(6):3148-51).

The screening strategy is to identify natural products and/or synthetic chemical entities that target the sordarin-sensitive step of fungal protein synthesis (elongation) but do not kill strains harboring the human eEF2 construct. Specifically, natural products are screened which show a differential activity between the human control and the other strains, although a 100% differential is not required in the initial discovery effort. It may be possible to observe hits solely against the fungal eEF2s, and/or the protozoal eEF2s. In sum, SWORDIII is designed to identify inhibitors of fungal and protozoal eEF2s to provide opportunities for developing anti-infective agents with a unique and selective mechanism of action.

Several *S*. *cerevisiae* mutants that exhibit resistance to varying levels of sordarin have been analyzed and the results demonstrated a functional linkage between specific regions of L10e/rpP0 and eEF2, with eEF2 identified as the specificity determinate. For this reason, the eEF2 encoding genes from different organisms and several species of pathogenic fungi have been cloned, characterized and expressed in *S. cerevisiae* at Merck Research Laboratory, as previously described (Microbiology 147:383-90, 2001). Site directed mutagenesis of the *C. parapsilosis* gene encoding eEF2 to create S521Y, N523S single mutants and the S521Y, N523S double mutant was accomplished by the PCR overlap extension technique (Gene 77:51-59, 1989). Mutant *S*. *cerevisiae* eEF2 genes encoding amino acid residues from *C. glabrata, C. guilliermondii, C. krusei, C. lusitaniae, C. parapsilosis, Cryptococcus neoformans, E. tenella, P. falciparum, C. parvum* or human EF2 were constructed by cassette mutagenesis as described (Protein Eng. 5:826-829, 1999).

Plasmid DNA from each fungal *EFT2* construct described above was used to transform the *S. cerevisiae Δeft1Δeft2* deletion strain YEFD12h to leucine proto-trophy. Colonies from the individual transformants were grown overnight in SC medium depleted of leucine and subsequently 1x 10⁶ cells were plated on 5FOA containing medium to evict the *pURA3-EFT1.* Transformants were also assayed for the predicted sordarin resistance phenotype by plating cells on medium containing sordarin at 5 µg/ml or 10 µg/ml. Plates were incubated at 29°C for approximately three days until colonies appeared. Growth inhibition assays to generate dose response curves were performed as previously described (J. Biol. Chem. 273:3148-3151, 1998). Therefore, SWORD III consists of an isogenic set of *S*. *cerevisiae* strains that differ only by the eEF2 construct they harbor.

### EXAMPLE 1

### Inoculate Seed Culture

The culture was maintained on agar plugs in vials containing sterile glycerol 10% stored at -80°C until ready for use. The seed culture was inoculated by aseptically transferring four agar plugs into a 250 ml Erlenmeyer flask containing 60ml seed medium of the following composition (in g/liter):
Corn steep powder, 2.5
Tomato paste, 40.0;
Oat flour, 10.0;
Glucose, 10.0 and
Trace elements solution, 10 ml/liter

The Trace elements solution consisted of the following components:
FeSO₄·7H₂O, 1.0 g/liter;
MnSO₄·4H₂O, 1.0 g/liter;
CuCl₂·2H₂O, 0.025 g/liter;
CaCl₂·2H₂O, 0.1 g/liter;
H₃BO₃, 0.056 g/liter;
(NH₄)₆MoO₂₄·4H₂O, 0.019 g/liter;
ZnSO₄·7H₂O, 0.2 g/liter;
dissolved in 0.6 N HCl.

Seed medium was prepared with distilled water. The pH was adjusted to 6.8 by adding NaOH, and the medium was dispensed into 250ml Erlenmeyer flasks and capped with cellulose plugs before being autoclaved at 121°C for 20 minutes.

The seed culture was incubated at 22°C on a gyratory shaker (220 rpm,) for 5 days prior to the inoculation of fermentation flasks.

### EXAMPLE 2

### Fermentation of the MF6856 in MV8 Medium

The MV8 production medium was formulated (per liter) as follows:

| | |
|---|---|
| Maltose | 75g |
| V8 Juice | 200ml |
| Soy Flour | 1g |
| L-Proline | 3g |
| MES | 16.2g |

The production medium was prepared with distilled water and no adjustment was made to the pH. It was dispensed into 500ml Erlenmeyer flasks (120ml per flask) and capped with cellulose plugs before being autoclaved at 121°C for 20 minutes. Fermentation flasks were inoculated with 4.8ml vegetative seed growth (4%) and were incubated at 22°C, 70% humidity on a gyratory shaker (220rpm) for 20 days. This fermentation broth is extracted directly with MEK.

### EXAMPLE 3

### Fermentation of the MF6856 in AD2M2 medium

The AD2M2 production medium was formulated (per liter) as follows:

Autoclave glucose in 1 liter distilled water. After autoclaving, add to other ingredients, which had been separately autoclaved in 1 liter of distilled water.

| K Elements | | Per liter | |
|---|---|---|---|
| Glucose | 300g | FeCl₃•6H₂O | 5.8g |
| Glyerol | 40g | MnSO₄•H₂O | 100mg |
| Yeast extract | 8g | CoCl₂•6H₂O | 20mg |
| NaNO₃ | 2g | CuSO₄•5H₂O | 15mg |
| Mono-Na Glutamate | 6g | Na₂MoO₄•2H₂O | 12mg |
| Na₂HPO₄ | 1g | ZnCl₂ | 20mg |
| MgSO₄•7H₂O | 2g | SnCl₂•2H₂O | 5mg |
| K-Elements* | 2ml | H₃BO₃ | 10mg |
| CaCO₃ | 16g | KCl | 20mg |
| | | HCl (conc) | 2ml |

The production medium was prepared with distilled water and adjustment was made to pH 7 before adding CaCO₃. It was dispensed into 500 ml Erlenmeyer flasks (220ml per flask) and capped with cellulose plugs before being autoclaved at 121°C for 20 minutes. Fermentation flasks were inoculated with 8.8ml vegetative seed growth (4%) and were poured in 1 liter Bellco bottles with 675 cc of vermiculite previously sterilized. They were incubated at 22°C, 70% humidity in rotation (4 rpm) for 20 days. The bottle with the fermentation broth is extracted directly with MEK.

### EXAMPLE 4

### Fermentation of the MF6864 in LSFM medium

The LSFM production medium was formulated (per liter) as follows:

| | |
|---|---|
| Glucose | 40 g |
| Glycerol | 18.7g |
| Tomato paste | 5 g |
| Ardamine | 5 g |
| Soy Bean meal | 5 g |
| (NH₄)₂SO₄ | 2 g |
| Sodium Citrate | 2 g |

The production medium was prepared with distilled water and adjustment was made to pH 7. It was dispensed into 500ml Erlenmeyer flasks (120ml per flask) and capped with cellulose plugs before being autoclaved at 121°C for 20 minutes. Fermentation flasks were inoculated with 4.8ml vegetative seed growth (4%) and were incubated at 22°C, 70% humidity on a gyratory shaker (220rpm) for 23 days. This fermentation broth is extracted directly with MEK.

### EXAMPLE 5A

### Isolation of the Compound of Formula I

A methyl ethyl ketone extract of a 12 liter solid fermentation of MF6856 in AD2M2 medium was concentrated to dryness in vacuo. The solid residue was dissolved in methanol (900 mL), diluted with H₂O (300 mL), and the solution adjusted to pH 6.6 with dilute NaOH. The resulting solution was clarified by filtration through celite and the filtrate applied at 10 mL/min to a column of BioRad AG4x4 (formate cycle, bed volume 200 mL). The column was washed with 75% MeOH/H₂O (900 mL) and then the compound of formula I was eluted with a solution of 100 mM sodium formate pH 4.5 in 75% MeOH/H₂O collecting 200 mL fractions. Compound of formula I eluted in fractions 2 and 3. Fractions 2 and 3 were combined, concentrated in vacuo to approximately 260 mL and the resulting solution adjusted to pH 3.1 with concentrated H₃PO₄. This solution was extracted 3 times with an equal volume of hexane:EtOAc (4:6). The organic layers were combined, washed with H₂O, washed with brine, dried over anhydrous Na₂SO₄, and concentrated *in vacuo* to yield 1.13g of crude compound of formula I.

The crude compound of formula I from above was further purified using high speed countercurrent chromatography (HSCCC). HSCCC separations were performed on P.C. Inc., multilayer coil planet centrifuge (Potomac, MD) equipped with a 300 mL, 1.68 mm I.D. coil. The column was first filled with a stationary phase consisting of CH₂Cl₂ + 0.04% trifluoroacetic acid. The crude compound of formula I from above was dissolved in stationary phase (10 mL) and injected onto the tail of the column. A mobile phase of aqueous 10 mM NH₄OH was pumped from tail to head of the coil at 3 mL/min with a coil rotation of 800 rpm. The pH of the coil effluent was monitored using a flow through pH electrode and 9.0 mL fractions were collected. The compound of formula I eluted in fractions 29-43. These fractions were combined and desalted as described above to yield crude compound of formula I, 296 mg.

Final purification of the compound of formula I was accomplished using RP HPLC. The crude compound of formula I from the HSCCC purification was dissolved in methanol (10 mL) and diluted with H₂O containing 0.1% formic acid (5 mL). This solution was subjected, in 3 equal runs, to preparative RP HPLC on C8, 15 um, 5 cm x 25 cm, using a mobile phase of CH₃CN/H₂O (6:4) containing 0.1% formic acid at a flow rate of 60 mL/min. The compound of formula I eluted at 18.7 min. Fractions containing the compound of formula I were combined, concentrated in vacuo to remove excess CH₃CN, and lyophilized to yield the compound of formula I, 135 mg.

Liquid fermentations of MF6856 were extracted with an equal portion of methanol and the solids removed by filtration through a celite pad. The filtrate (approx. 56 L) was adjusted to pH 3.1 with dilute H₂SO₄ and applied to a column of Mitsubishi SP207 Sepabeads (column volume = 2.5L) at 10 L /hr. The column was washed with 50% methanol/H₂O (8 L), and the compound of formula I eluted with methanol (6.5 L). The methanol eluate was diluted with 1850 mL H₂O and adjusted to pH 6.5 with dilute NaOH. This solution was further purified on a BioRad AG4x4 column (volume = 500 mL) as described above. Final purification of the compound of formula I from ion exchange rich cut was accomplished using RP HPLC (column = 7.5 cm x 25 cm) at a flow rate of 250 mL/min. using the conditions described above.

### EXAMPLE 6

### Identification of the Compound of Formula I

### Infrared Spectral Data

Under the infrared spectral analysis, the compound of formula I showed as a thin film on a ZnSe crystal, 1803, 1710, 1096 cm⁻¹.

### NMR Spectral Data

The 13C NMR spectra of the compound of formula I was recorded in CD₂C₁₂ at 125 MHz on a Varian Unity spectrometer at 25°C. Chemical shifts are given in ppm relative to tetramethyl silane (TMS) at zero ppm using the solvent peak at 53.8 ppm as internal standard.

13C NMR shifts of Compound I (1.0 mg in 0.125 mL of CD₂C₁₂ at 25°C): 205.1 (s), 178.9 (s), 176.2 (s), 172.9 (s), 148.8 (s), 131.1 (d), 108.2 (s), 98.7 (d), 83.0 (d), 76.2 (d), 74.9 (d), 74.1 (d), 72.9 (s), 70.1 (d), 65.9 (s), 62.1 (q), 59.2 (s), 46.9 (d), 42.1 (d), 41.6 (d), 34.2 (t), 32.5 (t), 32.4 (t), 31.4 (d), 29.8(t), 29.5(t), 29.3(t), 29.2 (t), 29.1 (t), 28.0 (d), 26.6 (t), 25.1 (t), 25.0 (t), 22.8 (q), 21.3 (q), 17.7 (q), 17.5 (q).

The ¹H NMR spectra of the compound of formula I was recorded in CD₂C₁₂ at 500 MHz on a Varian Unity spectrometer at 25°C. Chemical shifts are given in ppm relative to tetramethyl silane (TMS) at zero ppm using the solvent peak at 5.32 ppm as internal standard.

¹H NMR shifts of the compound of formula I (1.0 mg in 0.125 mL of CD₂C₁₂ at 25°C): 0.801 (d, 6.5), 0.967 (d, 6.5), 1.029 (d, 7.0), 1.25 (m), 1.314 (d, 6.5), 1.60 (m), 1.76 (m), 1.80 (m), 1.97 (m), 2.06 (m), 2.30 (m), 2.32 (m), 2.686 (brt, 3.0), 3.325 (d, 9.5), 3.474 (s), 3.544 (d, 9.5), 3.703 (d, 9.5), 3.714 (d, 1.00, 3.900 (d, 9.5), 4.566 (d, 1.0), 4.598 (dd, 4.0, 7.0), 6.088 (brt, 3.0).

### Mass Spectral Data

Analyzed by ESI-FTMS, [M+H] observed at m/z 691.3705, calculated for C₃₇H₅₅O₁₂, 691.3694.

### EXAMPLE 7

### Biological Function of the Compound of Formula I

### SwordIII Assay Data

*S. cerevisiae* strains expressing full length heterologous eEF2s under the *S. cerevisiae EFT2* promoter were generated as described in Shastry, et al. *Microbiology.* 2001, 147(2):383-90. IC₅₀ values were determined from growth inhibition assays in which cells were inoculated in SC medium-containing sordarin serially diluted 2 fold from 100-0.2 µg/ml, followed by incubation at 29°C for approximately 16 hours. See Table I.

**Table I**

| Strains | Sordarin | The Compound of Formula I |
|---|---|---|
| | IC50 (µg/ml) | |
| *S. cerevisiae*/SIII | 0.19 | 0.15 |
| *C. glabrata*/SIII | 6.25 | 5.0 |
| Human/SIII | >100 | >20 |
| *C. krusei*/SIII | >100 | 15 |
| *C.lusitaniae*/SIII | >100 | >20 |
| *C.neoformans*/SIII | >100 | 5.0 |
| *A.fumigatus*/SIII | >100 | 20 |
| *E.tenella*/SIII | >100 | >20 |

### In vitro translation assay data

*In vitro* translation assays were performed as described in Shastry, et al. *Microbiology,* 2001, 147(2):383-90. The IC₅₀ values are the average of three independent experiments. The lower limit of sensitivity is about 0.005 µg/ml. See Table II.

**Table II**

| Strains | Sordarin | The Compound of Formula I |
|---|---|---|
| | IC50 (µg/ml) | |
| *S. cerevisiae* | 0.006 | 0.006 |
| *C. albicans* | 0.006 | 0.006 |
| *C. glabrata* | 0.036 | 0.015 |
| *C.lusitaniae* | >100 | 0.5 |
| *C. parapsilosis* | >100 | 0.5 |
| *C. krusei* | >100 | 1 |
| *C.guilliermondi* | 0.01 | 0.006 |
| *C.neoformans* | 0.2 | 0.02 |
| *A.nidulans* | 2.6 | 0.6 |

### Assay Against Botrytis cinerea

3 milliliters of potato-dextrose agar (supplier: Difco) with drugs added at the concentrations noted below after autoclaving were allowed to set in the wells of a 24-well microtiter plate. 10 microliters of a spore suspension of *Botrytis cinerea* (ATCC 26943) was added to each well and the plate was incubated at 25°C. The potato-dextrose agar plates were dosed with A) sordarin and B) the compound of formula I at a) 0 µg/ml, b) 1 µg/ml, c) 2 µg/ml, d) 5 µg/ml, e) 10 µg/ml, and f) 20 µg/ml, then the plates were infected with a suspension of *Botrytis cinerea* (ATCC 26943) and photographed at 3 and 4 days. See Figure 1.

## Claims

1. A compound having the formula: 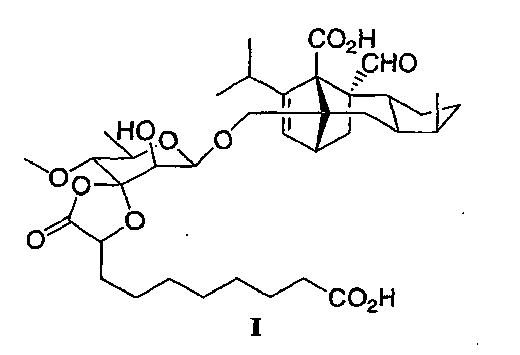 or a pharmaceutically or agriculturally acceptable salt thereof.

2. A pharmaceutical composition comprising a compound of claim 1 and a pharmaceutically acceptable carrier.

3. A pharmaceutical formulation comprising a combination of a compound of Claim 1 and a second therapeutic agent or its pharmaceutically acceptable salt.

4. The pharmaceutical formulation of Claim 3, wherein the second therapeutic agent is a compound selected from the group consisting of an azole, polyene, purine pyrimidine nucleotide inhibitor, pneumocandin derivative, echinocandin derivative, polyoxins, mannan inhibitors, bactericidal/permeability inducing protein products, elongation factor inhibitor, and immunomodulating agent.

5. The pharmaceutical formulation of Claim 4, wherein the second therapeutic agent is a compound selected from the group consisting of intraconazole, flucytosine, fluconazole, amphotericin B, and caspofungin.

6. An agrochemical composition, which comprises a compound of Claim 1 and an agriculturally acceptable carrier.

7. An agrochemical composition, which comprises a compound of Claim 1 and a second active ingredient selected from the group consisting of herbicides, insecticides, bactericides, nematocides, molluscicides, growth regulators, micronutrients, fertilizers, and fungicides.

8. Use of a compound of claim 1 for the manufacture of a medicament for the treatment or prevention of fungal infection in a mammal.

9. Use according to claim 8 wherein the treatment comprises administering to said mammal therapeutically effective amounts of a compound of Claim 1 and a second therapeutic agent selected from the group consisting of an azole, polyene, purine pyrimidine nucleotide inhibitor, pneumocandin derivative, echinocandin derivative, polyoxins, mannan inhibitors, bactsricidal/permeability inducing protein products, elongation factor inhibitor, and immunomodulating agent.

10. A method for controlling phytopathogenic fungi, which comprises administering to a plant in need of such control therapeutically effective amounts of a compound of Claim 1.

11. A method for controlling phytopathogenic fungi, which comprises administering to a plant in need of such control therapeutically effective amounts of a compound of Claim 1 and a second active ingredient selected from the group consisting of herbicides, insecticides, bactericides, nematocides, molluscicides, growth regulators, micronutrients, fertilizers, and fungicides.

12. Use of a compound of claim 1 for the manufacture of a medicament for inhibiting elongation factor 2 (EF2).

13. A culture of *Morinia sp.* that has been deposited with the American Type Culture Collection as ATCC No. PTA-3862.

14. A process of making the compound in claim 1 comprising the culturing and fermenting of ATCC No. PTA-3862.

15. A culture of *Cladosporium sp.* that has been deposited with the American Type Culture Collection as ATCC No. PTA-3861.

16. A process of making the compound in claim 1 comprising the culturing and fermenting of ATCC No. PTA-3861.

## Patentansprüche

1. Eine Verbindung mit der Formel: 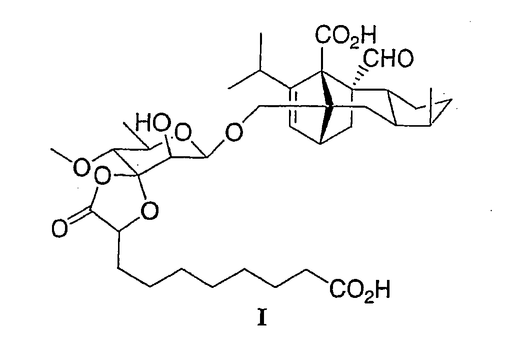 oder ein pharmazeutisch oder landwirtschaftlich annehmbares Salz davon.

2. Eine pharmazeutische Zusammensetzung, die eine Verbindung nach Anspruch 1 und einen pharmazeutisch annehmbaren Träger enthält.

3. Eine pharmazeutische Formulierung, die eine Kombination aus einer Verbindung nach Anspruch 1 und einem zweiten therapeutischen Mittel oder dessen pharmazeutisch annehmbarem Salz enthält.

4. Die pharmazeutische Formulierung nach Anspruch 3, wobei das zweite therapeutische Mittel eine Verbindung ist, ausgewählt aus der Gruppe, bestehend aus einem Azol, Polyen, Purin-Pyrimidin-Nukleotid-Inhibitor, Pneumocandin-Derivat, Echinocandin-Derivat, Polyoxinen, Mannan-Inhibitoren, bakteriziden/permeabilitäts-induzierenden Protein-Produkten, Elongationsfaktor-Inhibitor und immunmodulatorischem Mittel.

5. Die pharmazeutische Formulierung nach Anspruch 4, wobei das zweite therapeutische Mittel eine Verbindung ist, ausgewählt aus der Gruppe, bestehend aus Intraconazol, Flucytosin, Fluconazol, Amphotericin B und Caspofungin.

6. Eine agrochemische Zusammensetzung, die eine Verbindung nach Anspruch 1 und einen landwirtschaftlich annehmbaren Träger enthält.

7. Eine agrochemische Zusammensetzung, die eine Verbindung nach Anspruch 1 und einen zweiten Wirkstoff, ausgewählt aus der Gruppe, bestehend aus Herbiziden, Insektiziden, Bakteriziden, Nematoziden, Molluskiziden, Wachstumsreglern, Mikronährstoffen, Düngemitteln und Fungiziden, enthält.

8. Die Verwendung einer Verbindung nach Anspruch 1 zur Herstellung eines Medikaments zur Behandlung oder Prävention einer Pilzinfektion bei einem Säuger.

9. Die Verwendung gemäß Anspruch 8, wobei die Behandlung die Verabreichung therapeutisch wirksamer Mengen einer Verbindung nach Anspruch 1 und eines zweiten therapeutischen Mittels, ausgewählt aus der Gruppe, bestehend aus einem Azol, Polyen, Purin-Pyrimidin-Nukleotid-Inhibitor, Pneumocandin-Derivat, Echinocandin-Derivat, Polyoxinen, Mannan-Inhibitoren, bakteriziden/permeabilitäts-induzierenden Protein-Produkten, Elongationsfaktor-Inhibitor und immunmodulatorischem Mittel, an den Säuger umfasst.

10. Ein Verfahren zur Bekämpfung von phytopathogenen Pilzen, umfassend die Verabreichung therapeutisch wirksamer Mengen einer Verbindung nach Anspruch 1 an eine Pflanze, die eine solche Bekämpfung benötigt.

11. Ein Verfahren zur Bekämpfung von phytopathogenen Pilzen, umfassend die Verabreichung therapeutisch wirksamer Mengen einer Verbindung nach Anspruch 1 und eines zweiten Wirkstoffes, ausgewählt aus der Gruppe, bestehend aus Herbiziden, Insektiziden, Bakteriziden, Nematoziden, Molluskiziden, Wachstumsreglern, Mikronährstoffen, Düngemitteln und Fungiziden, an eine Pflanze, die eine solche Bekämpfung benötigt.

12. Die Verwendung einer Verbindung nach Anspruch 1 zur Herstellung eines Medikaments zur Inhibierung von Elongationsfaktor 2 (EF2).

13. Eine Kultur von *Morinia sp.,* die bei der American Type Culture Collection als ATCC Nr. PTA-3862 hinterlegt wurde.

14. Ein Verfahren zur Herstellung der Verbindung nach Anspruch 1, umfassend die Kultivierung und Fermentation von ATCC Nr. PTA-3862.

15. Eine Kultur von *Cladosporium sp.,* die bei der American Type Culture Collection als ATCC Nr. PTA-3861 hinterlegt wurde.

16. Ein Verfahren zur Herstellung der Verbindung nach Anspruch 1, umfassend die Kultivierung und Fermentation von ATCC Nr. PTA-3861.

## Revendications

1. Composé de formule: 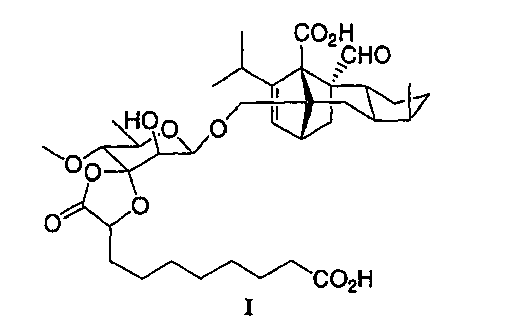 ou sel de ce composé acceptable en pharmacie ou en agriculture.

2. Composition pharmaceutique comprenant un composé selon la revendication 1 et un véhicule acceptable en pharmacie.

3. Formulation pharmaceutique comprenant une combinaison d'un composé selon la revendication 1 et d'un second agent thérapeutique ou de son sel acceptable en pharmacie.

4. Formulation pharmaceutique selon la revendication 3, dans laquelle le second agent thérapeutique est un composé choisi dans le groupe constitué par un azole, un polyène, un inhibiteur de nucléotide purine pyrimidine, un dérivé pneumocandine, un dérivé échinocandine, des polyoxines, des inhibiteurs de mannane, un inhibiteur du facteur d'élongation, des produits protéiques bactéricides/déclenchant la perméabilité et un agent d'immunomodulation.

5. Formulation pharmaceutique selon la revendication 4, dans laquelle le second agent thérapeutique est un composé choisi dans le groupe constitué par l'intraconazole, la flucytosine, le fluconazole, l'amphotéricine B et la caspofongine.

6. Composition agrochimique, qui comprend un composé selon la revendication 1 et un véhicule acceptable en agriculture.

7. Composition agrochimique, qui comprend un composé selon la revendication 1 et un second ingrédient actif choisi dans le groupe constitué par les herbicides, les insecticides, les bactéricides, les nématocides, les molluscicides, les régulateurs de croissance, les micronutriments, les engrais et les fongicides.

8. Utilisation d'un composé selon la revendication 1 pour la fabrication d'un médicament destiné au traitement ou à la prévention d'infection fongique chez un mammifère.

9. Utilisation selon la revendication 8 dans laquelle le traitement comprend l'administration audit mammifère de quantités thérapeutiquement efficaces d'un composé selon la revendication 1 et d'un second agent thérapeutique choisi dans le groupe constitué par un azole, un polyène, un inhibiteur de nucléotide purine pyrimidine, un dérivé pneumocandine, un dérivé échinocandine, des polyoxines, des inhibiteurs de mannane, un inhibiteur du facteur d'élongation, des produits protéiques bactéricides/déclenchant la perméabilité et un agent d'immunomodulation.

10. Procédé de lutte contre des champignons phytopathogènes, qui comprend l'administration, à un végétal ayant besoin d'une telle lutte, de quantités thérapeutiquement efficaces d'un composé selon la revendication 1.

11. Procédé de lutte contre des champignons phytopathogènes, qui comprend l'administration, à un végétal ayant besoin d'une telle lutte, de quantités thérapeutiquement efficaces d'un composé selon la revendication 1 et d'un second ingrédient actif choisi dans le groupe constitué par les herbicides, les insecticides, les bactéricides, les nématocides, les molluscicides, les régulateurs de croissance, les micronutriments, les engrais et les fongicides.

12. Utilisation d'un composé selon la revendication 1 pour la fabrication d'un médicament destiné à inhiber le facteur d'élongation 2 (EF2).

13. Culture de *Morinia sp.* qui a été déposée auprès de l'American Type Culture Collection sous la référence ATCC n° PTA-3862.

14. Procédé de fabrication du composé de la revendication 1 comprenant la culture et la fermentation de ATCC n° PTA-3862.

15. Culture de *Cladosporium sp.* qui a été déposée auprès de l'American Type Culture Collection sous la référence ATCC n° PTA-3861.

16. Procédé de fabrication du composé de la revendication 1 comprenant la culture et la fermentation de ATCC n° PTA-3861.
